(19) Europäisches Patentamt
European Patent Office
Office européen des brevets

(11) EP 1 083 169 B1

(12) EUROPÄISCHE PATENTSCHRIFT

(45) Veröffentlichungstag und Bekanntmachung des Hinweises auf die Patenterteilung:
**29.10.2003 Patentblatt 2003/44**

(51) Int Cl.[7]: **C07D 211/94**, C07D 211/00, C08F 4/00

(21) Anmeldenummer: **00118124.7**

(22) Anmeldetag: **28.08.2000**

# (54) Funktionalisierte Alkoxyamin-Initiatoren

Functionalized alkoxyamine initiators

Initiateurs fonctionalisés d'alcoxyamines

(84) Benannte Vertragsstaaten:
**DE ES FR GB IT NL**

(30) Priorität: **07.09.1999 DE 19942615**
**07.09.1999 DE 19942614**

(43) Veröffentlichungstag der Anmeldung:
**14.03.2001 Patentblatt 2001/11**

(73) Patentinhaber: **BAYER AG**
**51368 Leverkusen (DE)**

(72) Erfinder:
- **Melchiors, Martin, Dr.**
  **51373 Leverkusen (DE)**
- **Höcker, Hartwig, Prof. Dr.**
  **52076 Aachen (DE)**
- **Keul, Helmut, Dr.**
  **52074 Aachen (DE)**
- **Achten, Dirk**
  **52066 Aachen (DE)**

(56) Entgegenhaltungen:
**EP-A- 0 911 350** **WO-A-97/46593**

- **GRAVERT, DENNIS J. ET AL: "Soluble Supports Tailored for Organic Synthesis: Parallel Polymer Synthesis via Sequential Normal/Living Free Radical Processes" , J. AM. CHEM. SOC. (1998), 120(37), 9481-9495 XP002149956 * Seite 9484; Abbildung SCHEME2 ***
- **HAWKER, CRAIG J. ET AL: "Radical Crossover in Nitroxide Mediated "Living" Free Radical Polymerizations" , J. AM. CHEM. SOC. (1996), 118(46), 11467-11471 XP000960412 * Seite 11469; Abbildung SCHEME4 ***
- **HAWKER C J ET AL: "INITIATING SYSTEMS FOR NITROXIDE-MEDIATED LIVING FREE RADICAL POLYMERIZATIONS: SYNTHESIS AND EVALUATION" , MACROMOLECULES,US,AMERICAN CHEMICAL SOCIETY. EASTON, VOL. 29, NR. 16, PAGE(S) 5245-5254 XP000596748 ISSN: 0024-9297 * Seite 5249; Abbildung SCHEME4 ***
- **JAHN, ULLRICH: "Highly efficient generation of radicals from ester enolates by the ferrocenium ion. Application to selective.alpha.-oxygenation and dimerization reactions of esters" , J. ORG. CHEM. (1998), 63(21), 7130-7131 XP000960555 See entire doct.**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 083 169 B1

## Beschreibung

Die Erfindung betrifft ein neues Verfahren zur Herstellung funktionalisierter Alkoxyamin-Initiatoren, neue, nach diesem Verfahren hergestellte Alkoxyamin-Initiatoren auf (Meth)-acrylat-Basis, sowie ihre Verwendung zur Herstellung von Polymeren.

Die lebende radikalische Polymerisation stellt eine relativ junge Methode der kontrollierten radikalischen Polymerisation dar. Sie verbindet die Vorteile einer konventionellen radikalischen Polymerisation (einfaches Syntheseverfahren, breite Monomerbasis) mit denen einer lebenden Polymerisation (Polymere mit definiertem Aufbau, Molekulargewicht und -verteilung und Endgruppenfunktionalität). Das Ziel einer genauen Kontrolle der radikalischen Polymerisation wird hier durch einen reversiblen Kettenabbruch bzw. Blockierung ("end-capping") nach jedem Wachstumsschritt erreicht. Die Gleichgewichtskonzentration der polymerisationsaktiven ("living") Kettenenden ist dabei im Vergleich zur Gleichgewichtskonzentration der blockierten ("dormant") Kettenenden so gering, dass Abbruch- und Übertragungsreaktionen gegenüber der Wachstumsreaktion stark zurückgedrängt sind. Da das end-capping reversibel abläuft, bleiben alle Kettenenden "lebend" ("living"), sofern kein Abbruchreagenz vorhanden ist. Dies ermöglicht die Kontrolle des Molekulargewichts, einen niedrigen Polymolekularitätsindex und gezielte Funktionalisierung der Kettenenden durch Abbruchreagenzien.

Erste Ansätze für eine kontrollierte radikalische Polymerisation unter Verwendung von Tetraalkylthiuramdisulfiden werden bei Otsu et al. (Makromol. Chem., *Rapid Commun*. **1982,** *3*, 127-132) beschrieben.

Aus der US-A-4 581 429 sind Alkoxyamine bekannt, die durch Reaktion von linearen oder cyclischen Nitroxiden wie z.B. 2,2,6,6-Tetramethylpiperidin-1-oxyl (TEMPO) mit organischen Kohlenstoff-basierten Radikalen gebildet werden, sowie ein Verfahren zur Herstellung von Vinylpolymeren unter Verwendung dieser Verbindungen als Initiatoren. Bei Temperaturen >100°C kann die C-ON-Bindung reversibel unter Rückbildung des C-Radikals ("*active species*") und des stabilen Nitroxidradikals gespalten werden. Das Gleichgewicht liegt hierbei weit auf der Seite des Alkoxyamins ("*dormant species*").

Ergebnis dieser Reaktion ist eine geringe stationäre Radikalkonzentration, die bei der radikalischen Polymerisation von Vinylmonomeren dazu führt, dass bimolekulare Abbruchreaktionen gegenüber der unimolekularen Wachstumsreaktion kinetisch benachteiligt sind. So werden Nebenreaktionen weitgehend unterdrückt und eine "lebende" Reaktionsführung bei der radikalischen Polymerisation möglich. Die Verwendung von Alkoxyamin-Initiatoren, die zusätzlich funktionelle Gruppen tragen, ist nicht beschrieben.

Die Herstellung von Vinylpolymeren durch lebende radikalische Polymerisation ("**S**table **F**ree **R**adical **P**olymerization", SFRP) auf Basis von Alkoxyaminen beschreiben *Hawker et al*. (*J. Am. Chem. Soc*. **1994,** 116, 11185; *Macromolecules* **1995,** 28, 1993) sowie *Georges et al*. (Xerox Comp., US-A-5 322 912, US-A-5 401 804, US-A-5 412 047, US-A-5 449 724, WO 94/11412, WO 95/26987, WO 95/31484). Die Kohlenstoff-Radikale werden dabei durch Anlagerung von Radikalstartern (Peroxide, Azoinitiatoren) an radikalisch polymerisierbare Monomere hergestellt; diese Radikale werden dann in situ von TEMPO zu Alkoxyaminen abgefangen. Diese Alkoxyamine stellen die eigentlichen Initiatoren dar, da sie bei Temperaturen >100°C reversibel in Radikale gespalten werden und so die Polymerisation der zudosierten Monomere starten können. Die Verwendung funktionalisierter Alkoxyamin-Initiatoren würde so in einfacher Weise die Synthese von Polymeren mit gezielter Endgruppenfunktionalität ermöglichen, sofern die funktionellen Gruppen der Alkoxyamin-Initiatoren endständig im Polymer verbleiben.

Verschiedene Arbeitsgruppen haben sich mit der Synthese von Alkoxyamin-Initiatoren und im speziellen solcher funktioneller Alkoxyamin-Initiatoren für die SFRP von Vinylmonomeren befaßt. Die folgenden teilweise funktionalisierten Alkoxyamin-Initiatoren **I-IV** wurden dabei durch Reaktion einer radikalischen Spezies mit einem stabilen Nitroxidradikal, durch nucleophile Substitutionsreaktionen oder oxidative Addition dargestellt.

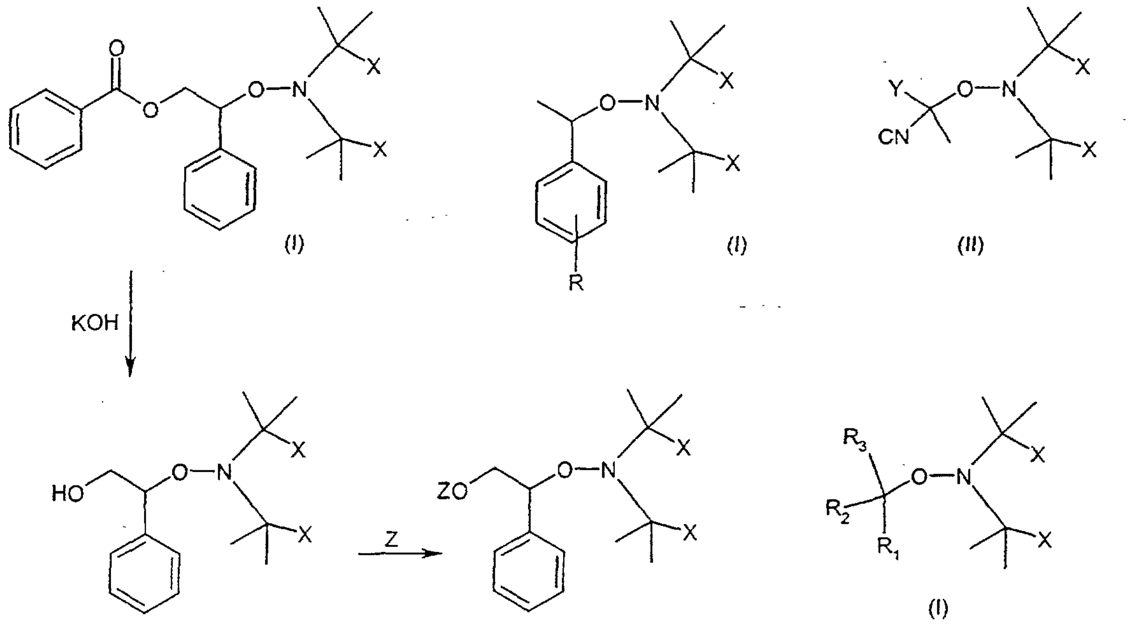


R = Br, COOEt, OMe, $CH_2OH$, Li, CHO
$R_1$, $R_2$, $R_3$ = verschiedene Reste, Ester, Alkyl, Acyl
X = Aryl, Alkyl (cyclisch oder offenkettig, funktionalisiert)
Y = verschiedene Reste, Alkyl, substituierte Alkyle, Aryle
Z = mit HO- zu einem Ester oder Ether reagierendes Reagenz

*Hawker et al*. (*Macromolecules* **1996,** 29, 5245-5254) entwickelten einen Syntheseweg zur Spezies I, bei dem vorgelegter Dibenzoylperoxid-Radikalstarter nach Addition an ein styrolisches Monomer von einem stabilen Nitroxidradikal abgefangen wird. Eine nachfolgende Hydrolyse der Esterfunktion liefert einen je nach Substitution des Nitroxidradikals mono- oder difunktionellen Alkoxyamin-Initiator. Dieses Verfahren zeigt niedrige Selektivität und liefert nur Ausbeuten von <<40 % an funktionalisierten Produkten.

Die Synthese von Alkoxyamin-Initiatoren des Typs **II** nach *Hawker et al*. (*Macromolecules* **1996,** 29, 5245-5254), *Yozo Miura et al*. (*Macromolecules* **1998,** 31, 4659-4661) und *Braslau et al*. (*Macromolecules* **1997,** 30, 6445-6450) gelingt mit radikalischen Initiatoren, die nach ihrem Zerfall aktivierte Wasserstoffatome aus geeigneten Substraten abstrahieren können. Typische Initiatoren sind Di-tertbutylperoxid, Di-tertbutylhyponitrit und Di-tertbutyldiperoxolat. Ausbeuten dieser Reaktionen liegen im Bereich von 40 bis 90 %. Durch Einsatz substituierter Aromaten (Br, COOEt, OMe) ist in weiteren Reaktionsschritten die Synthese hydroxyfunktioneller Alkoxyamin-Initiatoren möglich (*Yozo Miura et al. Macromolecules* **1998,** 31, 4659-4661). Die aufwendige mehrstufige Reaktionsführung über metallorganische Zwischenstufen machen diesen Prozess technisch kaum realisierbar und wirtschaftlich uninteressant.

Verbindungen des Typs III wurden von *Hawker et al*. (*Macromolecules* **1996,** 29, 5245-5254) durch Abfangen der bei dem Zerfall von Azoradikalstartem entstehenden Kohlenstoff-Radikale erhalten. Durch Einsatz von teuren funktionalisierten Azoradikalstartem ist so die Synthese funktionalisierter Alkoxyamin-Initiatoren möglich. Die Ausbeuten dieser Reaktion liegen bei <30%.

Alkoxyamin-Initiatoren des Typs **I** und **IV** wurden von Matyjaszewski et al. (*Macromolecules* **1998,** 31, 5955-5957) durch Umsetzung von benzylischen- bzw. anders aktivierten Bromverbindungen mit einem der ATRP (**A**tom **T**ransfer **R**adical **P**olymerisation) ähnlichen System aus $Cu^0/Cu^{2+}$ und einem substituierten Bipyridin in Anwesenheit eines stabilen Nitroxidradikals hergestellt. Dabei wird durch ATRA-Reaktion ((**A**tom **T**ransfer **R**adical **A**ddition) ein Kohlenstoff-Radikal unter Abstraktion des Bromatoms erzeugt, welches umgehend von dem Nitroxidradikal abgefangen wird. Die Ausbeuten für diese Reaktion liegen zwischen 76-92 %, funktionalisierte Alkoxyamin-Initiatoren wurden nicht hergestellt.

*Braslau et al*. (*Macromolecules* **1997,** 30, 6445-6450) berichten von der Synthese von Alkoxyamin-Initiatoren des Typs **II** und **IV** durch verschiedene anspruchsvolle mehrstufige mucleophile und oxidative oder photooxidative Additionsrouten mit Ausbeuten von 30-78 %. Funktionalisierte Alkoxyamin-Initiatoren wurden nicht hergestellt.

Eine weitere Route zu Alkoxyamin-Initiatoren nach *Bergbreiter et al*. (*Macromolecules* **1998,** 31, 6380-6382) führt ausgehend von allylischen Aminen in Ausbeuten von 10 bis 74 % zu N-Allyloxyaminen. Funktionelle Alkoxyamin-Initiatoren wurden nicht hergestellt.

Mit keiner der beschriebenen Methoden lassen sich in technisch einfacher Weise und in hohen Ausbeuten funktionalisierte Alkoxyamine mit 1 oder 2 funktionellen Gruppen herstellen, die zu einer weiteren Umsetzung oder Vernetzung mit in der Lackchemie gebräuchlichen funktionellen Gruppen fähig sind.

Aufgabe der vorliegenden Erfindung war daher die Bereitstellung eines Verfahrens zur Herstellung von funktionellen Initiatoren des Typs In-OH und/oder Y-In-OH, das die obengenannten Nachteile des Standes der Technik nicht aufweist, wobei "In" einen substituierten Kohlenwasserstoffrest darstellt, der zum Start einer SFRP befähigt ist und Y eine funktionelle Gruppe, die zu einer weiteren Umsetzung oder Vernetzung mit in der Lackchemie gebräuchlichen funktionellen Gruppen fähig ist, darstellt.

Diese Aufgabe konnte gelöst werden durch ein einstufiges Verfahren zur Herstellung von Alkoxyamin-Initiatoren der allgemeinen Formel (I)

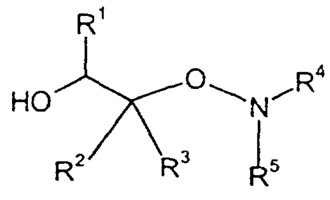

(I),

wobei

$R^1$, $R^2$, $R^3$ unabhängig voneinander H, $C_1$-$C_{20}$-(Cyclo)alkyl, $C_6$-$C_{24}$-Aryl, Halogen, CN, $C_1$-$C_{20}$-(Cyclo)alkylester oder -amid, $C_6$-$C_{24}$-Arylester oder -amid sein können, und die Einheit $R^4R^5NO$ einer der Formeln (III)-(VI) entspricht,

dadurch gekennzeichnet, dass

A) ein radikalisch polymerisierbares Monomer der allgemeinen Formel (M)

$$HR^1C = CR^2R^3,$$

wobei

$R^1$, $R^2$, $R^3$ unabhängig voneinander H, $C_1$-$C_{20}$-(Cyclo)alkyl, $C_6$-$C_{24}$-Aryl, Halogen, CN, $C_1$-$C_{20}$-(Cyclo)alkylester oder -amid, $C_6$-$C_{24}$-Arylester oder -amid sein können, mit

B) einem Radikale produzierenden System aus

B1) $Fe^{2+}$,

B2) wasserstoffperoxid

B3) einem cyclischen oder acyclischen Nitroxid der Formeln (III)-(VI)

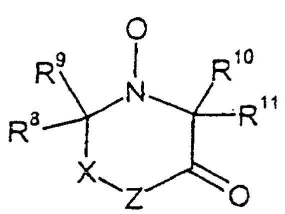

(III),

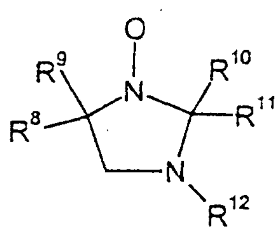

(IV),

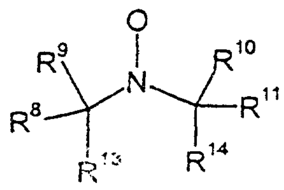

(V),

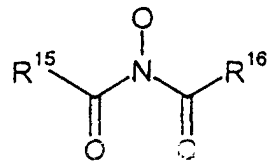

(VI),

wobei in

**Formel (III) bis (VI):**

$R^8$, $R^9$, $R^{10}$ und $R^{11}$ unabhängig voneinander $C_1$-$C_{20}$-(Cyclo)alkyl- oder $C_6$-$C_{24}$-Arylreste bzw. $C_7$-$C_{24}$-aliphatisch/aromatische Kohlenwasserstoffreste sind, weiche zusätzlich Cyanogruppen, Ethergruppen, Amidgruppen oder OH-Gruppen enthalten können und auch Teil einer Ringstruktur sein können, wobei X auch $CH_2$ oder C = O sein kann, Z für O oder N-$R^{12}$ stehen kann und $R^{12}$, $R^{15}$ und $R^{16}$ unabhängig voneinander für H oder einen $C_1$-$C_{20}$-(Cyclo)alkyl- oder $C_6$-$C_{24}$-Arylrest bzw. einen $C_7$-$C_{24}$-aliphatisch/aromatischen Kohlenwasserstoffrest stehen, wobei diese Substituenten auch Teil einer Ringstruktur sein können und in

**Formel (V):**

$R^8$, $R^9$, $R^{10}$ und $R^{11}$ die obengenannte Bedeutung haben und die Reste $R^{13}$ und $R^{14}$ unabhängig voneinander $C_1$-$C_{20}$-(Cyclo)alkyl-, $C_6$-$C_4$-Aryl- bzw. $C_7$-$C_{24}$-aliphatisch/aromatische Kohlenwasserstoffreste bedeuten und auch Teil einer Ringstruktur sein können, wobei $R^{13}$ und/oder $R^{14}$ funktionelle Gruppen, ausgewählt aus substituierten oder unsubstituierten Phenyl-, Cyano-, Ether-, Hydroxy-, Nitro-, Dialkyloxyphosphonyl- oder carbonyltragenden Gruppen enthalten können und die Gruppen $CR^8R^9R^{13}$ und/oder $CR^{10}R^{11}R^{14}$ auch Teil eines aromatischen Ringsystems sein können oder eine Phenylgruppe bilden können,

in einem Lösungsmittel umgesetzt werden.

Das erfindungsgemäße Verfahren ermöglicht die einfache, einstufige Herstellung der funktionalisierten Alkoxyamin-Initiatoren in hohen Ausbeuten ausgehend von preiswerten, einfach zugänglichen Grundchemikalien.

Als radikalisch polymerisierbare Monomere (M) der Komponente (A) können prinzipiell alle aus dem Stand der Technik bekannten radikalisch polymerisierbaren, gegebenenfalls substituierten Olefine eingesetzt werden. Als Substituenten für die Olefine kommen z.B. in Frage:

- H,
- lineare oder verzweigte Alkylreste mit 1 bis 20 Kohlenstoffatomen, die gegebenenfalls auch weitere Substituenten tragen können,
- $\alpha,\beta$-ungesättigte lineare oder verzweigte Alkenyl- oder Alkinylreste, die auch Heteroatome wie z.B. O, N oder S im Ring und gegebenenfalls weitere Substituenten tragen können,
- gegebenenfalls substituierte Aryl- oder Heteroarylreste,
- Halogen, CN, $CF_3$, COOR, COR.

Die radikalisch polymerisierbare Doppelbindung der Monomeren (M) kann auch Teil eines Ringes sein, wie z.B. bei cyclischen Olefinen oder olefinisch ungesättigten cyclischen Anhydriden oder Imiden.

Bevorzugt eingesetzte Monomere sind (Meth)acrylsäureester von $C_1$-$C_{20}$-Alkoholen, Acrylnitril, Cyanoacrylsäureester von $C_1$-$C_{20}$-Alkoholen, Maleinsäurediester von $C_1$-$C_6$-Alkoholen, Maleinsäureanhydrid, Vinylpyridine, Vinyl (alkylpyrrole), Vinyloxazole, Vinyloxazoline, Vinylthiazole, Vinylimidazole, Vinylpyrimidine, Vinylketone, Styrol oder Styrolderivate, die in a-Stellung einen $C_1$-$C_6$-Alkylrest oder Halogen tragen und bis zu 3 weitere Substituenten am aromatischen Ring tragen.

Besonders bevorzugt werden Methylacrylat, Methylmethacrylat, Butylacrylat, Butylmethacrylat, 2-Ethylhexylacrylat, Cyclohexylmethacrylat, Isobornylmethacrylat, Maleinsäureanhydrid oder Styrol eingesetzt.

In keinem der oben im Stand der Technik beschriebenen Dokumente für die Synthese von Alkoxyamin-Initiatoren ist der Gebrauch von Wasserstoffperoxid als Radikallieferant erwähnt.

Wasserstoffperoxid ist als Reinsubstanz und in wäßriger Lösung (z.B. 30 % Perhydrol) eine thermodynamisch metastabile Verbindung. Durch Katalysatoren (z.B. fein verteilte Metalle, Braunstein, Staubteilchen, Nichtmetallionen wie $I^-$, $IO_3^-$, OH- oder Metallionen wie $Fe^{2+}$, $Fe^{3+}$, $Cu^{2+}$) wird die Zufallsgeschwindigkeit des Wasserstoffperoxids auch bei Raumtemperatur stark erhöht. Hydroxyl-Radikale können aus Wasserstoffperoxid gezielt durch thermische Zersetzung des Wasserstoffperoxids oder durch Einelektronen-Redoxreaktionen des Wasserstoffperoxids mit einem geeigneten Elektronendonator erzeugt werden. Typische Verbindungen sind z.B. Natriumhydrogensulfit, leicht enolisierbare Carbonylverbindungen wie Ascorbinsäure, Hydroxyaceton und Metallionen wie $Fe^{2+}$, $Ti^{3+}$[3] und $Cu^{1+}$. Die Reaktion von $Fe^{2+}$ mit Wasserstoffperoxid zu HO·-Radikalen, die zur Oxidation organischer Verbindungen benutzt werden können, ist unter dem Namen Fentons's Reagenz bekannt geworden:

$$Fe^{2+} + HO\text{-}OH \rightarrow Fe^{3+} + HO\bullet + HO^{-}.$$

Das bei der Redox-Reaktion entstehende HO- kann ebenfalls den Peroxidzerfall einleiten.

In Gegenwart gesättigter organischer Verbindungen HR kann das Hydroxyradikal unter H-Abstraktion reagieren. Darüberhinaus vermögen sich die HO· Radikale auch an Mehrfachbindungen ungesättigter organischer Verbindungen zu addieren.

Im erfindungsgemäßen Verfahren zur Herstellung eines Alkoxyamin-Initiators der Formel (I) addiert an die sich ein OH-Radikal an eine C=C-Doppelbindung des radikalisch polymerisierbaren Monomeren (M) und führt so eine Hydroxylgruppe in den Alkoxyamin-Initiator ein.

**B3** Bei der Komponente B3 handelt es sich um als Radikalfänger wirkende Verbindungen, die das aus den Komponenten A und (B1+B2) gebildete Monomerradikal vor einer Weiterreaktion im Sinne einer Polymerisation abfangen.

Erfindungsgemäß eingesetzt werden als Komponente B3 Nitroxide einer der folgenden Formeln (III) bis (VI)

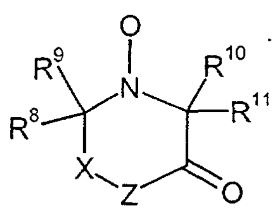

(III),

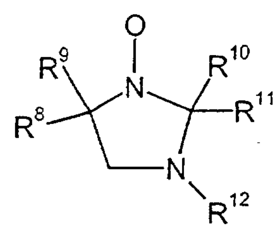

(IV),

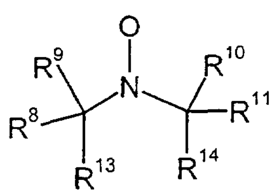

(V),

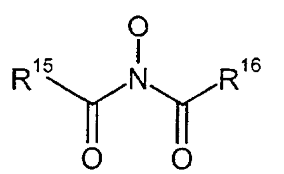

(VI),

einsetzt, wobei in

**Formel (III) bis (VI):**

$R^8$, $R^9$, $R^{10}$ und $R^{11}$ unabhängig voneinander $C_1$-$C_{20}$-(Cyclo)alkyl- oder $C_6$-$C_{24}$-Arylreste bzw. $C_7$-$C_{24}$-aliphatisch/aromatische Kohlenwasserstoffreste sind, welche zusätzlich Cyanogruppen, Ethergruppen, Amidgruppen oder OH-Gruppen enthalten können und auch Teil einer Ringstruktur sein können, wobei X auch $CH_2$ oder C = O sein kann, Z für O oder N-$R^{12}$ stehen kann und $R^{12}$, $R^{15}$ und $R^{16}$ unabhängig voneinander für H oder einen $C_1$-$C_{20}$-(Cyclo)alkyl- oder $C_6$-$C_{24}$-Arylrest bzw. einen $C_7$-$C_{24}$-aliphatisch/aromatischen Kohlenwasserstoffrest stehen, wobei diese Substituenten auch Teil einer Ringstruktur sein können und in

**Formel (V):**

$R^8$, $R^9$, $R^{10}$ und $R^{11}$ die obengenannte Bedeutung haben und die Reste $R^{13}$ und $R^{14}$ unabhängig voneinander $C_1$-$C_{20}$-(Cyclo)alkyl-, $C_6$-$C_{24}$-Aryl- bzw. $C_7$-$C_{24}$aliphatisch/aromatische Kohlenwasserstoffreste bedeuten und auch Teil einer Ringstruktur sein können, wobei $R^{13}$ und/oder $R^{14}$ funktionelle Gruppen, ausgewählt aus substituierten oder unsubstituierten Phenyl-, Cyano-, Ether-, Hydroxy-, Nitro-, Dialkyloxyphosphonyl- oder carbonyltragenden Gruppen enthalten können und die Gruppen $CR^8R^9R^{13}$ und/oder $CR^{10}R^{11}R^{14}$ auch Teil eines aromatischen Ringsystems sein können oder eine Phenylgruppe bilden können.

Bevorzugt werden als Komponente B3 solche der allgemeinen Formel (VII) verwendet:

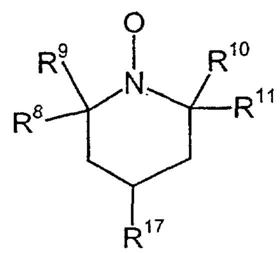

(VII),

worin

$R^8$, $R^9$, $R^{10}$ und $R^{11}$ die obengenannte Bedeutung haben, und $R^{17}$ entweder Wasserstoff oder eine funktionelle Gruppe Y ist, die zu einer weiteren Umsetzung oder Vernetzung mit in der Lackchemie gebräuchlichen funktionellen Gruppen fähig ist; Y kann z.B. eine Hydroxygruppe, Aminogruppe oder Epoxygruppe sein.

Besonders bevorzugt werden als Komponente B3 Nitroxide der Strukturformel **VII** mit $R^8 = R^9 = R^{10} = R^{11} = CH_3$ und $R^{17}$ = H, OH oder $NH_2$ eingesetzt.

Zur Durchführung des erfindungsgemäßen Verfahrens zur Herstellung der erfindungsgemäßen funktionalisierten Alkoxyamin-Initiatoren legt man eine Lösung der Komponenten A, B1 und B3 in einem Lösemittel oder Lösemittelgemisch vor und dosiert die radikalbildende Komponente B2 unter Rühren langsam zu. B2 wird dabei bevorzugt als wässrige Lösung in einem 0,1- bis 20fachen molaren Überschuß bezogen auf B3 zudosiert. B 1 wird äquimolar, bevorzugt aber in einem bis zu 20 %igen molaren Überschuß, bezogen auf B3, eingesetzt. Das Monomer A wird, bezogen auf B3, in einem 0,2- bis 20-fachen molaren Überschuß eingesetzt. Überschüssige Anteile der eingesetzten Komponenten werden nach beendeter Umsetzung destillativ oder extraktiv wieder entfernt. Die Umsetzung findet bei Temperaturen zwischen 0°C und 150°C, bevorzugt 40°C bis 100°C, statt. Sie kann an der Luft oder in einer Schutzgasatmosphäre durchgeführt werden; bevorzugt verwendet man eine Schutzgasatmosphäre (z.B. Stickstoff oder Argon). Der pH-Wert der Reaktionslösung kann gegebenenfalls mit Substanzen wie z.B. $NaHCO_3$ auf einen Bereich von 5 bis 7 eingestellt werden. Bei bestimmten funktionellen Gruppen (z.B. Y = $NH_2$) kann es vorteilhaft sein, die funktionellen Gruppen während der beschriebenen Reaktion mit einer Schutzgruppe zu versehen (z.B. Schützen von Aminogruppen als Acetamide; später Freisetzung der Aminofunktion durch Hydrolyse mit einer Base); für Y = OH ist die Verwendung von Schutzgruppen aber nicht notwendig.

Bei der erfindungsgemäßen Herstellung des Alkoxyamin-Initiators der Formel (I) werden als Lösungsmittel Wasser, Alkohole, bevorzugt Methanol, Ethanol oder Isopropanol, Ether, bevorzugt Diethylether, Oligoethylenglykole oder THF, Carbonylverbindungen, bevorzugt Acetaldehyd, Aceton oder Methylethylketon oder beliebige Gemische der genannten Lösemittel eingesetzt.

Die erfindungsgemäß hergestellten funktionalisierten Alkoxyamin-Initiatoren haben die allgemeine Formel (I)

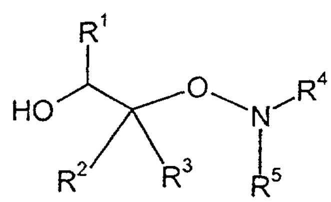

(I),

wobei

$R^1$, $R^2$, $R^3$, $R^4$ und $R^5$ die obengenannte Bedeutung haben, und worin mindestens einer der Reste $R^4$, $R^5$ eine funktionelle Gruppe Y enthalten kann, die zu einer weiteren Umsetzung oder Vernetzung mit

in der Lackchemie gebräuchlichen funktionellen Gruppen fähig ist.

Insbesondere können nach dem erfindungsgemäßen Verfahren neue Alkoxyamin-Initiatoren der allgemeinen Formel (II)

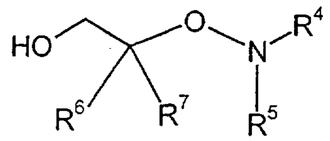

(II),

in welcher

$R^4$, $R^5$, $R^6$ und $R^7$ die obengenannte Bedeutung haben, auf Basis von Acrylat- und Methacrylat-Monomeren, wie sie in der Lacktechnologie üblicherweise in Polyacrylat-(Co)polymeren eingesetzt werden, in einfacher Weise und hoher Ausbeute hergestellt werden. Diese neuen Alkoxyamin-Initiatoren der Formel (II) weisen mindestens eine OH-Gruppe auf; bevorzugt enthalten sie in mindestens einem der Reste $R^4$, $R^5$ noch eine weitere funktionelle Gruppe Y, die zu einer weiteren Umsetzung oder Vernetzung mit in der Lackchemie gebräuchlichen funktionellen Gruppen fähig ist.

Besonders bevorzugt sind die neuen Alkoxyamin-Initiatoren der Formel (IIb)

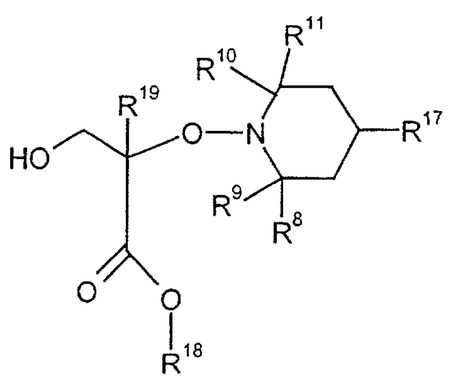

(IIb),

worin

$R^8$, $R^9$, $R^{10}$ und $R^{11}$ die bei Formel (VII) genannte Bedeutung haben,

$R^{17}$ für Wasserstoff oder eine funktionelle Gruppe Y steht, die zu einer weiteren Umsetzung oder Vernetzung mit in der Lackchemie gebräuchlichen funktionellen Gruppen fähig ist und

$R^{18}$ für eine (cyclo)aliphatische Alkylgruppe mit 1 bis 20 Kohlenstoffatomen steht,

$R^{19}$ für H oder $CH_3$ steht.

Insbesondere ist in Formel (IIb) $R^8 = R^9 = R^{10} = R^{11} = CH_3$ und

Y eine der funktionellen Gruppen OH, $NH_2$ oder NHR, die über die Nitroxid-Komponente B3 in den Alkoxyamin-Initiator eingeführt werden.

Die erfindungsgemäß hergestellten Alkoxyamin-Initiatoren der Formel (I) können z.B. als Radikalstarter zur Herstellung von Vinyl(co)polymeren bzw. -oligomeren, insbesondere von solchen mit funktionellen Gruppen als Endgruppen, verwendet werden.

**Beispiele**

Erfindungsgemäße Herstellung der funktionalisierten Alkoxyamin-Initiatoren: Alle Angaben in % beziehen sich auf das Gewicht.

**Beispiel 1**

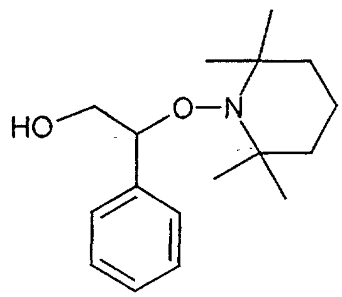

HOST: 1-Phenyl-1-(2',2',6'6'-tetramethyl-1'-piperidinyloxy)-2-hydroxyethan

In einem 1 l Zweihalskolben werden unter Stickstoffatmosphäre zu einer homogenen Lösung von 1 mol Styrol (104,14 g), 0,1 mol TEMPO (2,2,6,6-Tetramethylpiperidin-1-oxyl, 15,6 g) in 300 ml Methanol 0,12 mol Fe(II)$SO_4$ · $7H_2O$ (33,36 g) und 18,5 g $NaHCO_3$ (0,24 mol) gegeben und unter starkem Rühren auf 40°C erwärmt. Zu der roten Reaktionslösung werden 50 ml 30 % $H_2O_2$ Lösung in 50 ml Methanol innerhalb von 5 h langsam zugetropft.

Nach Verschwinden der Rotfärbung - Zeichen eines vollständigen Umsatzes von TEMPO - werden das entstandene Fe(III)-Hydroxyd, nicht umgesetztes Fe(II)$SO_4$ · $7H_2O$ und $NaHCO_3$ durch Filtrieren aus dem Reaktionsansatz entfernt. Das Filtrat wird am Rotationsverdampfer bei RT von Methanol befreit. Durch Extraktion mit Ether werden das Produkt (HOST) und Styrol von der wässrigen Phase getrennt. Die etherische Lösung wird über $Na_2SO_4$ getrocknet. Das Trockenmittel wird abfiltriert und Ether und Styrol werden am Rotationsverdampfer bei 40°C abgezogen. Man erhält den monofunktionalisierten Alkoxyamin-initiator in 71 % Ausbeute.

**Beispiel 2**

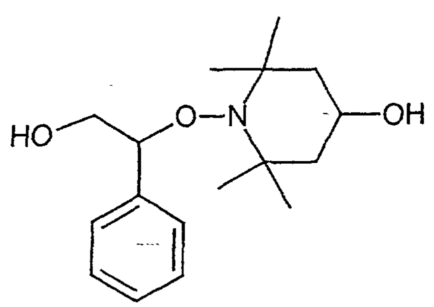

HOSTOH: 1-Phenyl-1- (4' -hydroxy -2',2',6',6' -tetramethyl-1'-piperidinyloxy) -2 -hydroxyethan

In einem 1 l Zweihalskolben werden unter Stickstoffatmosphäre zu einer homogenen Lösung von 1 mol Styrol (104,14 g), 0,1 mol 4-HO-TEMPO (4-Hydroxy-2,2,6,6-tetramethylpiperidin-1-oxyl, 17,33 g) in 300 ml Methanol 0,12 mol Fe(II)$SO_4$ · $7H_2O$ (33,36 g) und 18,5 g $NaHCO_3$ (0,24 mol) gegeben und unter starkem Rühren auf 40°C erwärmt.

Zu der roten Reaktionslösung werden 50 ml 30 % $H_2O_2$ Lösung in 50 ml Methanol innerhalb von 5 h langsam zugetropft.

Nach Verschwinden der Rotfärbung - Zeichen eines vollständigen Umsatzes von 4-HO-TEMPO - werden das entstandene Fe(III)-Hydroxyd, nicht umgesetztes Fe(II)$SO_4$ · $7H_2O$ und $NaHCO_3$ durch Filtrieren aus dem Reaktionsansatz entfernt. Das Filtrat wird am Rotationsverdampfer bei RT von Methanol befreit. Durch Extraktion mit Ether werden das Produkt (HOSTOH) und Styrol von der wässrigen Phase getrennt. Die etherische Lösung wird über $Na_2SO_4$ getrocknet. Das Trockenmittel wird abfiltriert und Ether und Styrol werden am Rotationsverdampfer bei 40°C abgezogen. Man erhält den difunktionalisierten Alkoxyamin-Initiator in 95 % Ausbeute.

**Beispiel 3**

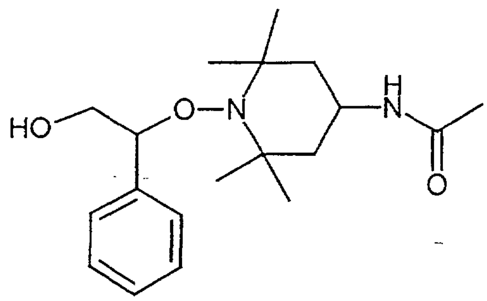

HOSTNHCOCH$_3$: 1-Phenyl-1- (4' -acetylamido -2',2',6',6'-tetramethyl-1'-piperidinyloxy)-2-hydroxyethan

In einem 0,5 l Zweihalskolben werden unter Stickstoffatmosphäre zu einer homogenen Lösung von 0,5 mol Styrol (52,07 g), 0,03 mol 4-Acetylamido-TEMPO (4-Acetylamido-2,2,6,6-tetramethylpiperidin-1-oxyl, 6,03 g) in 150 ml Methanol 0,04 mol Fe(II)$SO_4$ · $7H_2O$ (11,1 g) und 6,7 NaHCO3 (0,08 mol) gegeben und unter starkem Rühren auf 40°C erwärmt. Zu der roten Reaktionslösung werden 20 ml 30 % $H_2O_2$ Lösung in 20 ml Methanol innerhalb von 5 h langsam zugetropft.

Nach Verschwinden der Rotfärbung - Zeichen eines vollständigen Umsatzes von 4-Acetylamido-TEMPO - werden das entstandene (Fe(III)-Hydroxyd, nicht umgesetztes Fe(II)$SO_4$ · $7H_2O$ und $NaHCO_3$ durch Filtrieren aus dem Reaktionsansatz entfernt. Das Filtrat wird am Rotationsverdampfer bei RT von Methanol befreit. Durch Extraktion mit Ether werden das Produkt (HOSTNHCOCH$_3$) und Styrol von der wässrigen Phase getrennt. Die etherische Lösung wird über $Na_2SO_4$ getrocknet. Das Trockenmittel wird abfiltriert und Ether und Styrol werden am Rotationsverdampfer bei 40°C abgezogen. Man erhält den monofunktinalisierten Alkoxyamin-Initiator in 85 % Ausbeute.

**Beispiel 4**

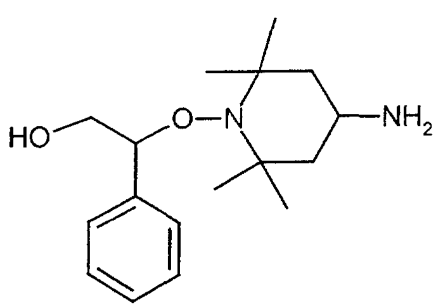

HOSTNH$_2$: 1-Phenyl-1-(4'-Amino-2',2',6',6'-tetramethyl-1'-piperidinyloxy ) -2-hydroxyethan

In einem 200 ml Schlenkkolben wird unter Stickstoffatmosphäre 0,0018 mol HOSTNHCOCH$_3$ (0,6 g) zu einer Lösung von 10 g KOH in 20 ml Wasser und 20 ml Ethylenglykol gegeben. Die Lösung wird für 12 h auf 100°C erhitzt

und im Anschluß 5 mal mit jeweils 20 ml Ether extrahiert. Die etherische Phase wird 2 mal mit 30 ml einer wässrigen HCl Lösung mit einem pH von 3 gewaschen. Das Produkt befindet sich nun in der sauren wässrigen Phase. Diese wird mit 10 g KOH versetzt und erneut mit Ether extrahiert. Die etherische Phase wird über $Na_2SO_4$ getrocknet und filtriert, der Ether am Rotationsverdampfer entfernt und das Produkt im Hochvakuum getrocknet. Der difunktionalisierte Alkoxyamin-Initiator $HOSTNH_2$ wird in 59 % Ausbeute als weißer Feststoff erhalten.

**Beispiel 5**

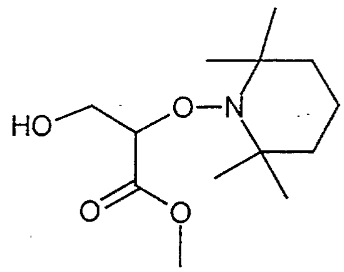

HOMAT: 3 -Hydroxy -2 - (2',2',6',6'-tetramethyl-1'-piperidinyloxy ) -propionsäuremethylester

In einem 1 l Zweihalskolben werden unter Stickstoffatmosphäre zu einer homogenen Lösung von 1 mol Methylacrylat (86,09 g), 0,1 mol TEMPO (2,2,6,6-Tetramethylpiperidin-1-oxyl, 15,6 g) in 300 ml Methanol 0,12 mol Fe(II) $SO_4 \cdot 7H_2O$ (33,36 g) und 18,5 g $NaHCO_3$ (0,24 mol) gegeben und unter starkem Rühren auf 40°C erwärmt. Zu der roten Reaktionslösung werden 50 ml 30 % $H_2O_2$ Lösung in 50 ml Methanol innerhalb von 5 h langsam zugetropft.
Nach Verschwinden der Rotfärbung - Zeichen eines vollständigen Umsatzes von TEMPO- werden das entstandene Fe(III)-Hydroxyd, nicht umgesetztes $Fe(II)SO_4 \cdot 7H_2O$ und $NaHCO_3$ durch Filtrieren aus dem Reaktionsansatz entfernt. Das Filtrat wird am Rotationsverdampfer bei RT von Methanol befreit. Durch Extraktion mit Ether werden das Produkt (HOMAT) und Methylacrylat von der wässrigen Phase getrennt. Die etherische Lösung wird über $Na_2SO_4$ getrocknet. Das Trockenmittel wird abfiltriert und Ether und Methylacrylat werden am Rotationsverdampfer bei 40°C abgezogen. Man erhält den monofunktionalisierten Alkoxyamin-Initiator in 76 % Ausbeute.

**Beispiel 6**

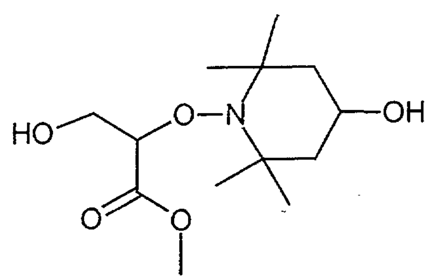

HOMATOH: 3 -Hydroxy -2 - (4' -hydroxy -2',2',6',6' -tetramethyl-1'-piperidinyloxy ) -propionsäuremethylester

In einem 1 l Zweihalskolben werden unter Stickstoffatmosphäre zu einer homogenen Lösung von 1 mol Methylacrylat (86,09 g), 0,1 mol 4-HO-TEMPO (4-Hydroxy-2,2,6,6-tetramethylpiperidin-1-oxyl, 17,33 g) in 300 ml Methanol 0,12 mol $Fe(II)SO_4 \cdot 7H_2O$ (33,36 g) und 18,5 g $NaHCO_3$ (0,24 mol) gegeben und unter starken Rühren auf 40°C erwärmt. Zu der roten Reaktionslösung werden 50 ml 30 % $H_2O_2$ Lösung in 50 ml Methanol innerhalb von 5 h langsam zugetropft.
Nach Verschwinden der Rotfärbung - Zeichen eines vollständigen Umsatzes von 4-HO-TEMPO - werden das entstandene Fe(III)-Hydroxyd, nicht umgesetztes $Fe(II)SO_4 \cdot 7H_2O$ und $NaHCO_3$ durch Filtrieren aus dem Reaktionsansatz entfernt. Das Filtrat wird am Rotationsverdampfer bei RT von Methanol befreit. Durch Extraktion mit Ether werden das Produkt (HOMATOH) und Methylacrylat von der wässrigen Phase getrennt. Die etherische Lösung wird über $Na_2SO_4$ getrocknet. Das Trockenmittel wird abfiltriert und Ether und Methylacrylat werden am Rotationsverdampfer bei 40°C abgezogen. Man erhält den difunktionalisierten Alkoxyamin-Initiator in 60 % Ausbeute.

**Beispiel 7**

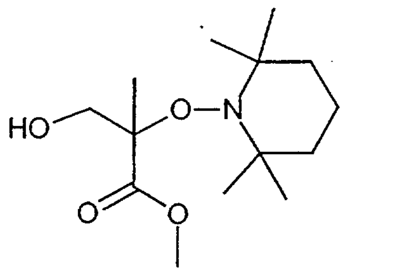

HOMMAT: 3-Hydroxy-2-methyl-2-(2',2',6',6'-tetramethyl-1'-piperidinyloxy) -propionsäuremethylester

In einem 0,5 l Zweihalskolben mit Magnetkern werden unter Stickstoffatmosphäre zu einer homogenen Lösung von 0,5 mol Methylmethacrylat (50 g), 0,05 mol TEMPO (2,2,6,6-Tetramethylpiperidin-1-oxyl, 7,8 g) in 150 ml Methanol 0,06 mol $Fe(II)SO_4 \cdot 7H_2O$ (16,68 g) und 9,25 g $NaHCO_3$ (0,12 mol) zugegeben und unter starkem Rühren auf 40°C erwärmt. Zu der roten Reaktionslösung werden 25 ml 30 % $H_2O_2$ Lösung in 25 ml Methanol innerhalb von 5 h langsam zugetropft.

Nach Verschwinden der Rotfärbung - Zeichen eines vollständigen Umsatzes von TEMPO- werden das entstandene Fe(III)-Hydroxyd, nicht umgesetztes $Fe(II)SO_4 \cdot 7H_2O$ und $NaHCO_3$ durch Filtrieren aus dem Reaktionsansatz entfernt. Das Filtrat wird am Rotationsverdampfer bei RT von Methanol befreit. Durch Extraktion mit Ether werden das Produkt (HOMMAT) und Methylmethacrylat von der wässrigen Phase getrennt. Die etherische Lösung wird über $Na_2SO_4$ getrocknet. Das Trockenmittel wird abfiltriert und Ether und Methylmethacrylat werden am Rotationsverdampfer bei 40°C abgezogen. Man erhält den monofunktionalisierten Alkoxyamin-Initiator in 66 % Ausbeute.

**Beispiel 8**

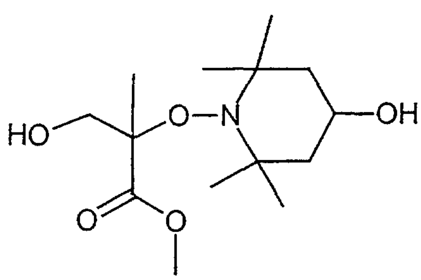

HOMMATOH: 3 -Hydroxy -2 -Methyl -2 - (4' -hydroxy -2',2',6',6' -tetramethyl-1'-piperidinyloxy)-propionsäuremethylester

In einem 0,5 1 Zweihalskolben werden unter Stickstoffatmosphäre zu einer homogenen Lösung von 0,5 mol Methylmethacrylat (50 g), 0,05 mol 4-HO-TEMPO (4-Hydroxy-2,2,6,6-tetramethylpiperidin-1-oxyl, 8,66 g) in 150 ml Methanol 0,06 mol $Fe(II)SO_4 \cdot 7H_2O$ (16,68 g) und 9,25 g $NaHCO_3$ (0,12 mol) gegeben und unter starkem Rühren auf 40°C erwärmt. Zu der roten Reaktionslösung werden 25 ml 30 % $H_2O_2$ Lösung in 25 ml Methanol innerhalb von 5 h langsam zugetropft.

Nach Verschwinden der Rotfärbung - Zeichen eines vollständigen Umsatzes von 4-HO-TEMPO - werden das

entstandene Fe(III)-Hydroxyd, nicht umgesetztes Fe(II)$SO_4 \cdot 7H_2O$ und $NaHCO_3$ durch Filtrieren aus dem Reaktionsansatz entfernt. Das Filtrat wird am Rotationsverdampfer bei RT von Methanol befreit. Durch Extraktion mit Ether werden das Produkt (HOMMATOH) und Methylmethacrylat von der wässrigen Phase getrennt. Die etherische Lösung wird über $Na_2SO_4$ getrocknet. Das Trockenmittel wird abfiltriert und Ether und Methylmethacrylat werden am Rotationsverdampfer bei 40°C abgezogen. Man erhält den difunktionalisierten Alkoxyamin-Initiator HOMMATOH nach Umkristallisieren aus Methylenchlorid in 37 % Ausbeute als farblose Kristalle.

Die Produkte der Beispiele 1 bis 8 können gegebenenfalls durch Umfällen oder Umkristallisieren weiter gereinigt werden.

## Patentansprüche

1. Verfahren zur Herstellung von Alkoxyamin-Initiatoren der allgemeinen Formel (I)

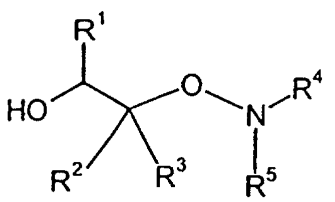

(I),

wobei

$R^1$, $R^2$, $R^3$ unabhängig voneinander H, $C_1$-$C_{20}$-(Cyclo)alkyl, $C_6$-$C_{24}$-Aryl, Halogen, CN, $C_1$-$C_{20}$-(Cyclo)alkylester oder -amid, $C_6$-$C_{24}$-Arylester oder -amid sein können, und die Einheit $R^4R^5NO$ einer der Formel (III) - (VI) entspricht,

**dadurch gekennzeichnet, dass**

A) ein radikalisch polymerisierbares Monomer der allgemeinen Formel (M)

$$HR^1C = CR^2R^3,$$

wobei

$R^1$, $R^2$, $R^3$ unabhängig voneinander H, $C_1$-$C_{20}$-(Cyclo)alkyl, $C_6$-$C_{24}$-Aryl, Halogen, CN, $C_1$-$C_{20}$-(Cyclo)alkylester oder -amid, $C_6$-$C_{24}$-Arylester oder -amid sein können, mit

B) einem Radikale produzierenden System aus

B1) $Fe^{2+}$,

B2) Wasserstoffperoxid

B3) einem cyclischen oder acyclischen Nitroxid der Formeln (III)-(VI)

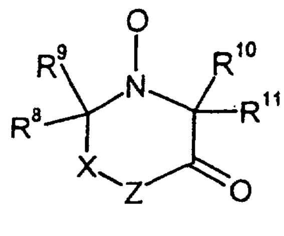

(III),

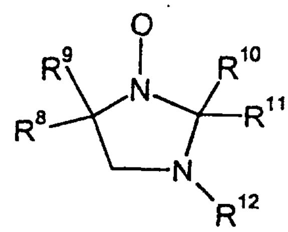

(IV),

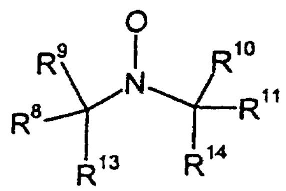

(V),

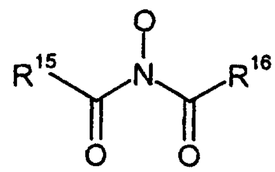

(VI),

wobei in

**Formel (III) bis (VI):**

$R^8$, $R^9$, $R^{10}$ und $R^{11}$ unabhängig voneinander $C_1$-$C_{20}$-(Cyclo)alkyl- oder $C_6$-$C_{24}$-Arylreste bzw. $C_7$-$C_{24}$-aliphatisch/aromatische Kohlenwasserstoffreste sind, welche zusätzlich Cyanogruppen, Ethergruppen, Amidgruppen oder OH-Gruppen enthalten können und auch Teil einer Ringstruktur sein können, wobei X auch $CH_2$ oder C = O sein kann, Z für O oder N-$R^{12}$ stehen kann und $R^{12}$, $R^{15}$ und $R^{16}$ unabhängig voneinander für H oder einen $C_1$-$C_{20}$-(Cyclo)alkyl- oder $C_6$-$C_{24}$-Arylrest bzw. einen $C_7$-$C_{24}$-aliphatisch/aromatischen Kohlenwasserstoffrest stehen, wobei diese Substituenten auch Teil einer Ringstruktur sein können und in

**Formel (V):**

$R^8$, $R^9$, $R^{10}$ und $R^{11}$ die obengenannte Bedeutung haben und die Reste $R^{13}$ und $R^{14}$ unabhängig voneinander $C_1$-$C_{20}$-(Cyclo)alkyl-, $C_6$-$C_{24}$-Aryl- bzw. $C_7$-$C_{24}$-aliphatisch/aromatische Kohlenwasserstoffreste bedeuten und auch Teil einer Ringstruktur sein können, wobei $R^{13}$ und/oder $R^{14}$ funktionelle Gruppen, ausgewählt aus substituierten oder unsubstituierten Phenyl-, Cyano-, Ether-, Hydroxy-, Nitro-, Dialkyloxyphosphonyl- oder carbonyltragenden Gruppen enthalten können und die Gruppen $CR^8R^9R^{13}$ und/oder $CR^{10}R^{11}R^{14}$ auch Teil eines aromatischen Ringsystems sein können oder eine Phenylgruppe bilden können, in einem Lösungsmittel umgesetzt werden.

2. Verfahren zur Herstellung von Alkoxyamin-Initiatoren gemäß Anspruch 1, **dadurch gekennzeichnet, dass** in Formel (I) $R^1$ = H, ist $R^2$ = H oder ein $C_1$-$C_{20}$-(Cyclo)alkyl-Rest und $R^3$ ein linearer oder verzweigter $C_1$-$C_{24}$-(Cyclo) alkyloxocarbonyl-Rest oder CN ist.

3. Verfahren zur Herstellung von Alkoxyamin-Initiatoren gemäß Anspruch 1 oder 2, **dadurch gekennzeichnet, dass** man als Komponente A Methylacrylat, Methylmethacrylat, Butylacrylat, Butylmethacrylat, 2-Ethylhexylacrylat, Cyclohexylmethacrylat, Isobornylmethacrylat, Maleinsäureanhydrid oder Styrol einsetzt.

4. Verfahren zur Herstellung von Alkoxyamin-Initiatoren gemäß einem der Ansprüche 1-3, **dadurch gekennzeichnet, dass** man als Komponente B3 Nitroxide der Formel (VII) einsetzt

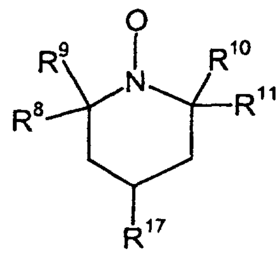

(VII),

worin
$R^8$, $R^9$, $R^{10}$ und $R^{11}$ die in Anspruch 1 genannte Bedeutung haben, und $R^{17}$ für Wasserstoff oder eine funktionelle Gruppe Y, die OH, $NH_2$, NHR oder Epoxy ist, steht.

**5.** Verfahren zur Herstellung von Alkoxyamin-Initiatoren gemäß Anspruch 4, **dadurch gekennzeichnet, dass** man als Komponente B3 Nitroxide der Strukturformel (VII) mit $R^8 = R^9 = R^{10} = R^{11} = CH_3$ und $R^{17}$ = H, OH oder $NH_2$ einsetzt.

**Claims**

**1.** Process for the preparation of alkoxyamine initiators of the general formula (I)

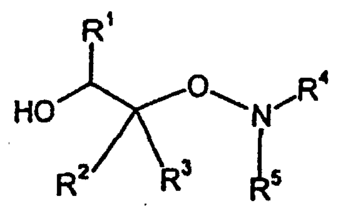

(I),

wherein

$R^1$, $R^2$ and $R^3$ independently of one another can be H, $C_1$-$C_{20}$-(cyclo)alkyl, $C_6$-$C_{24}$-aryl, halogen, CN, $C_1$-$C_{20}$-(cyclo)alkyl ester or -amide or $C_6$-$C_{24}$-aryl ester or -amide, and the unit $R^4R^5NO$ corresponds to one of the formulae (III)-(VI),

**characterized in that**

A) a monomer which can be polymerized by free radicals, of the general formula (M)

$$HR^1C = CR^2R^3,$$

wherein

$R^1$, $R^2$ and $R^3$ independently of one another can be H, $C_1$-$C_{20}$-(cyclo)alkyl, $C_6$-$C_{24}$-aryl, halogen, CN, $C_1$-$C_{20}$-(cyclo)alkyl ester or - amide or $C_6$-$C_{24}$-aryl ester or -amide,

are reacted with

B) a system, which produces free radicals, of

B1) $Fe^{2+}$,

B2) hydrogen peroxide,

B3) a cyclic or acyclic nitroxide of the formulae (III)-(VI)

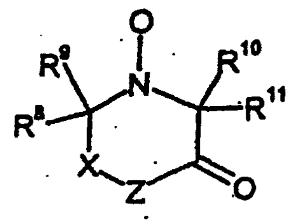

(III),

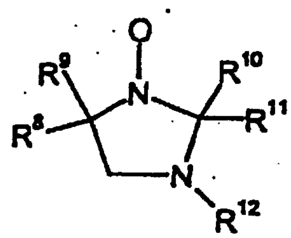

(IV),

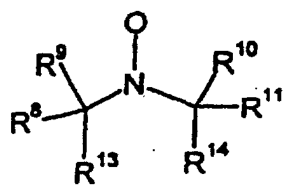

(V),

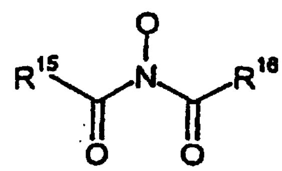

(VI),

wherein in

**formula (III) to (VI):**

$R^8$, $R^9$, $R^{10}$ and $R^{11}$ independently of one another are $C_1$-$C_{20}$-(cyclo)alkyl or $C_6$-$C_{24}$-aryl radicals or $C_7$-$C_{24}$-aliphatic/aromatic hydrocarbon radicals, which can additionally contain cyano groups, ether groups, amide groups or OH groups and can also be part of a ring structure, wherein X can also be $CH_2$ or C = O, Z can represent O or N-$R^{12}$ and $R^{12}$, $R^{15}$ and $R^{16}$ independently of one another represent H or a $C_1$-$C_{20}$-(cyclo)alkyl or $C_6$-$C_{24}$-aryl radical or a $C_7$-$C_{24}$-aliphatic/aromatic hydrocarbon radical, wherein these substituents can also be part of a ring structure, and in

**formula (V):**

$R^8$, $R^9$, $R^{10}$ and $R^{11}$ have the abovementioned meaning and the radicals $R^{13}$ and $R^{14}$ independently of one another denote $C_1$-$C_{20}$-(cyclo)alkyl, $C_6$-$C_{24}$-aryl or $C_7$-$C_{24}$-aliphatic/aromatic hydrocarbon radicals and can also be part of a ring structure, wherein $R^{13}$ and/or $R^{14}$ can contain functional groups chosen from substituted or unsubstituted phenyl, cyano, ether, hydroxyl, nitro, dialkyloxyphosphonyl or carbonyl-carrying groups and the groups $CR^8R^9R^{13}$ and/or $CR^{10}R^{11}R^{14}$ can also be part of an aromatic ring system or can form a phenyl group

in a solvent.

**2.** Process for the preparation of alkoxyamine initiators according to claim 1, **characterized in that** in formula (I) $R^1$ = H, $R^2$ = H or a $C_1$-$C_{20}$-(cyclo)alkyl radical and $R^3$ is a linear or branched $C_1$-$C_{24}$-(cyclo)alkyloxocarbonyl radical or CN.

**3.** Process for the preparation of alkoxyamine initiators according to claim 1 or 2, **characterized in that** methyl acrylate, methyl methacrylate, butyl acrylate, butyl methacrylate, 2-ethylhexyl acrylate, cyclohexyl methacrylate, isobornyl methacrylate, maleic anhydride or styrene is employed as component A.

**4.** Process for the preparation of alkoxyamine initiators according to one of claims 1 - 3, **characterized in that** as component B3, nitroxides of the formula (VII) are employed

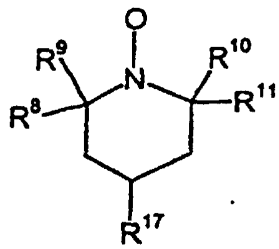

(VII),

wherein
$R^8$, $R^9$, $R^{10}$ and $R^{11}$ have the meaning given in claim 1 and $R^{17}$ represents hydrogen or a functional group Y, which is OH, $NH_2$, NHR oder epoxy.

**5.** Process for the preparation of alkoxyamine initiators according to claim 4, **characterized in that** as component B3, nitroxides of the structural formula (VII) where $R^8 = R^9 = R^{10} = R^{11} = CH_3$ and $R^{17}$ = H, OH or $NH_2$ are employed.

## Revendications

**1.** Procédé pour la préparation d'inducteurs du type alcoxyamine répondant à la formule générale (I)

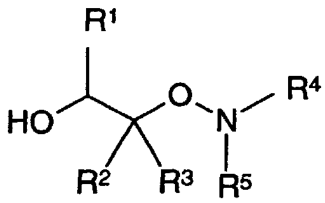

(I),

dans laquelle

$R^1$, $R^2$, $R^3$ représentent chacun, indépendamment les uns des autres, H, un groupe (cyclo)alkyle en $C_1$-$C_{20}$, aryle en $C_6$-$C_{24}$, un halogène, un groupe CN, un groupe ester ou amide (cyclo)alkylique en $C_1$-$C_{20}$, ester ou amide arylique en $C_6$-$C_{24}$, et le motif $R^4R^5NO$ répond à l'une des formules (III) à (VI),

**caractérisé en ce que** l'on fait réagir dans un solvant

A) un monomère apte à la polymérisation radicalaire, de formule générale (M)

$$HR^1C = CR^2R^3,$$

dans laquelle

$R^1$, $R^2$, $R^3$ représentent chacun, indépendamment les uns des autres, H, un groupe (cyclo)alkyle en $C_1$-$C_{20}$, aryle en $C_6$-$C_{24}$, un halogène, un groupe CN, un groupe ester ou amide (cyclo)alkylique en $C_1$-$C_{20}$, ester ou amide arylique en $C_6$-$C_{24}$,

avec
B) un système producteur de radicaux libres consistant lui-même en

B1) $Fe^{+2}$,
B2) le peroxyde d'hydrogène
B3) un nitroxyde cyclique ou acyclique de formule (III) à (VI)

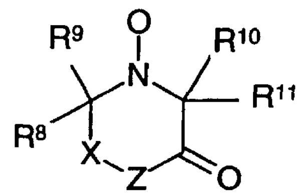

(III),

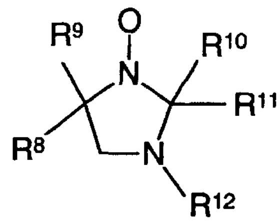

(IV),

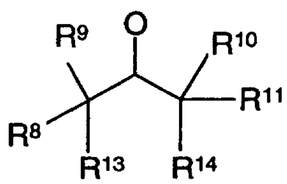

(V),

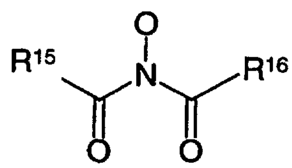

(VI),

pour lesquelles
dans les formules (III) à (VI) :
$R^8$, $R^9$, $R^{10}$ et $R^{11}$ représentent chacun, indépendamment les uns des autres, un groupe (cyclo)alkyle en $C_1$-$C_{20}$ ou aryle en $C_6$-$C_{24}$ ou un radical hydrocarboné aliphatique/aromatique en $C_7$-$C_{24}$, ces groupes pouvant contenir en outre des groupes cyano, éther, amide ou OH ou pouvant faire partie d'une structure cyclique, X peut également représenter $CH_2$ ou C=O, Z représente O ou N-$R^{12}$ et $R^{12}$, $R^{15}$ et $R^{16}$ représentent chacun, indépendamment les uns des autres, H ou un groupe (cyclo)alkyle en $C_1$-$C_{20}$ ou aryle en $C_6$-$C_{24}$ ou un radical aliphatique/aromatique en $C_7$-$C_{24}$, ces substituants pouvant également faire partie d'une structure cyclique et
dans la formule (V) :
$R^8$, $R^9$, $R^{10}$ et $R^{11}$ ont les significations indiquées ci-dessus et $R^{13}$ et $R^{14}$ représentent chacun, indépendamment l'un de l'autre, un groupe (cyclo)alkyle en $C_1$-$C_{20}$, aryle en $C_6$-$C_{24}$ ou un radical hydrocarboné aliphatique/aromatique en $C_7$-$C_{24}$, et qui peuvent également faire partie d'une structure cyclique, $R^{13}$ et/ou $R^{14}$ représentent des groupes fonctionnels choisis parmi les groupes phényle substitué ou non, cyano, éther, hydroxy, nitro, dialkyloxyphosphonyle et les groupes portant des fonctions carbonyle, et les groupes $CR^8R^9R^{13}$ et/ou $CR^{10}R^{11}R^{14}$ pouvant également faire partie d'un système cyclique aromatique ou pouvant former un groupe phényle.

2. Procédé pour la préparation des inducteurs du type alcoxyamine selon la revendication 1, **caractérisé en ce que**, dans la formule (I), $R^1$ = H, $R^2$ = H ou radical (cyclo)alkyle en $C_1$-$C_{20}$ et $R^3$ = radical (cyclo)alkyloxocarbonyle à chaîne droite ou ramifiée en $C_1$-$C_{24}$, ou CN.

3. Procédé pour la préparation des inducteurs du type alcoxyamine selon la revendication 1 ou 2, **caractérisé en ce que** pour le composant A, on utilise l'acrylate de méthyle, le méthacrylate de méthyle, l'acrylate de butyle, le méthacrylate de butyle, l'acrylate de 2-éthylhexyle, le méthacrylate de cyclohexyle, le méthacrylate d'isobornyle, l'anhydride maléique ou le styrène.

4. Procédé pour la préparation des inducteurs du type alcoxyamine selon l'une des revendications 1 à 3, **caractérisé en ce que**, pour le composant B3, on utilise des nitroxydes de formule (VII)

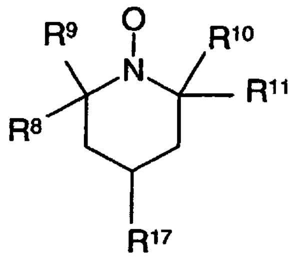

(VII),

dans laquelle
$R^8$, $R^9$, $R^{10}$ et $R^{11}$ ont les significations indiquées dans la revendication 1 et $R^{17}$ représente l'hydrogène ou un groupe fonctionnel Y consistant en un groupe OH, $NH_2$, NHR ou époxy.

**5.** Procédé pour la préparation des inducteurs du type alcoxyamine selon la revendication 4, **caractérisé en ce que**, pour le composant B3, on utilise des nitroxydes de formule de structure (VII) dans laquelle $R^8=R^9=R^{10}=R^{11}=CH_3$ et $R^{17}$=H, OH ou $NH_2$.